# EUROPEAN PATENT APPLICATION

(11) **EP 3 620 063 A1**
(43) Date of publication of application: **11.03.2020**
(21) Application number: 18192916.7
(22) Date of filing: 06.09.2018
(51) Int. Cl.: A23K 10/37, C13K 1/06, C12P 19/02, C12P 19/14, A23K 10/38, A23K 50/10, A23K 50/30, A23K 50/90

(54) **METHOD FOR THE PRODUCTION OF FEED STARCH SYRUP FROM STARCH-CONTAINING PLANT RAW MATERIALS**

(71) Applicant: Animal Food Solution Latvija, SIA, 5016 Keguma nov. (LV)
(72) Inventor: Aksenov, Vladimir, LV-5016 Keguma nov. (LV)
(74) Representative: Vitina, Maruta

(57) **Abstract**

The present invention relates to the production of feed additives for productive animals. More specifically, the present invention relates to the production of concentrated feed additives with high contents of glucose and other easily digestible carbohydrates, namely, feed starch syrup, from the starch-containing grain and grain legumes by the enzymatic method. The invention makes it possible to increase the environmental safety and stability of the process, ensures complete starch processing, and affords a product with a high content of simple carbohydrates suitable for long-term storage without additional treatment. The method for the production of feed starch syrup from starch-containing plant raw materials comprises the cavitation treatment of water in a disperser, introduction of ground starch-containing plant raw material at a weight ratio of water to starch-containing raw material of 1 : 0.4-0.6 and liquefying amylolytic enzymes, gelation and liquefaction of plant starch; introduction of the second part of the starch-containing raw material pretreated with IR radiation in an amount sufficient for reaching the total weight ratio of water to starch-containing raw material of 1 : 0.9-1.1 and liquefying amylolytic enzymes, gelation and liquefaction of plant starch, introduction of saccharifying amylolytic enzymes, and saccharification of starch.

## Description

The present invention relates to the production of feed additives for productive animals. More specifically, the present invention relates to the production of concentrated feed additive, namely, feed starch syrup with high contents of glucose and other easily digestible carbohydrates from starch-containing raw materials, namely, grain and grain legumes by the enzymatic method.

The known methods for the production of liquid carbohydrate-containing feed products from grain raw materials include the stages of preparation of aqueous grain suspensions by mixing the pretreated starch-containing (grain) raw material with water, heating the suspensions to the gelation stage, enzymatic liquefaction of the gelated reaction mass, and enzymatic saccharification of the liquefied suspension [RU2085590, C 13K1/06, Jul. 27, 1997; RU2285725, C 13K1/06, Nov. 16, 2004; RU2596753, A 23K40/00, Sep. 10, 2016].

The simplest method for the preliminary processing of grain is its mechanical grounding - crushing. However, when the crushed grain is further treated in dispersers when obtaining sugar feed additives in an aqueous medium, stable emulsions form as a result of effective deagglomeration and disintegration of the components of the treated mass in these devices, and the protein components of granulated grain cause strong stable foaming during the processing. Because of strong foaming, the apparatus is not completely filled and some part of the product is lost. The foaming hinders the preparation of concentrated carbohydrate suspensions from granulated grain.

The preliminary infrared (IR) irradiation of the grain and leguminous raw materials leads to protein denaturation, which reduces the foaming of the reaction mass during further processing. However, IR irradiation leads to an incomplete destruction of the grain surface shell; it mainly decreases the strength properties of the shell, while the grain itself is not disintegrated. In a process with high concentrations of IR irradiated grain in aqueous medium, the grain is completely disintegrated only as a result of passing many times through the working parts of the disperser. Therefore, impassable blocks can form at the initial stages of grain processing, stopping the process for the production of concentrated feed syrups.

The concentration of easily digestible carbohydrates in the end product (feed starch syrup) depends on the starch content in the grain and on the amount of grain in the aqueous grain suspension. In similar processes, the grain concentration is generally no more than 30-33% (here and below, weight percent is meant) because at the gelation stage of the enzymatic hydrolysis of grain starch, during the heating to the point of the start of gelation, the viscosity of the reaction mixture abruptly and rapidly increases by a factor of 220-300. To prevent the abrupt increase in viscosity, which leads to a loss of fluidity of the reaction mass and terminates the process, strong acids and/or liquefying amylolytic enzymes are used. At more than 30% grain in the reaction mixture, however, there is high probability of the loss of fluidity, and liquefaction cannot occur because of the high viscosity of the medium and inability of enzymes to liquefy the starch components amylose and amylopectin. Therefore, the water to grain ratio in these processes is generally 2 : 1, or 3 : 1, or (sometimes) 4 : 1 (for example, [RU 2285725, C 13K1/06, Nov. 16, 2004]).

Another problem concerning feed sugar products with low (less than 28-30%) contents of carbohydrates (glucose, maltose) is their short shelf life. The shelf life of these syrups or molasses is no more than 12-18 h above 25°C and a few days at lower temperatures because carbohydrate mixtures are a good breeding ground for the development of pathogenic and opportunistic pathogenic microflora. Acetic fermentation occurs under aerobic conditions, and alcoholic fermentation takes place under anaerobic conditions. To increase the shelf life, the concentration of carbohydrates in molasses is increased by evaporating the resulting solutions. The carbohydrate-containing solutions are evaporated at atmospheric pressure at temperatures above 100°C. At these temperatures, however, caramelization occurs, with carbohydrates actively interacting with one another; another reaction is the Maillard reaction of carbohydrates with amino acids present in the grain; these reactions deteriorate the qualitative characteristics of the product and lead to a decrease in the carbohydrate content and formation of compounds dangerous to animals. Evaporation of water in vacuum is performed at temperatures of up to 70°C; however, the above reactions cannot be excluded. During vacuum evaporation, some useful components, including carbohydrates, are carried away with steam. Evaporation of water from carbohydrate solutions is a long, energy-consuming process that requires special expensive equipment.

The closest prior art of the proposed method is the "Method for the production of feed molasses" [RU 2 265 364 (A 23 K 1/00, C 13K1/06, Dec. 18, 2003], which includes the following operations: preliminary heating of water to 35-40°C in a separate vessel, mixing of ground grain with warm water in a cavitator, adjustment of pH of the medium with hydrochloric acid and soda ash, gradual feeding of ground grain while introducing enzymes after the introduction of half of the amount of ground grain, cavitation treatment of the grain suspension (40 min), storage of the reaction mass without treatment (30 min), second cavitation treatment of the grain mass (40 min), transferring the resulting suspension into a vessel for saccharification, saccharification of the suspension in a separate container with a stirring device, iodine test to determine the end of the process, and evaporation of feed molasses in a vacuum evaporator.

This method is multioperational and technologically complicated. It is implemented using several devices: cavitator, container for heating water, container for the preparation of acid solutions, fermenter with a stirrer for starch saccharification, and vacuum evaporator. Another disadvantage of the method is the use of hydrochloric acid and soda ash in starch hydrolysis, which significantly reduces the environmental safety of the process, requires the use of additional equipment, increases the material costs of the production of molasses, and increases the content of inorganic impurities in the end product. The use of strong acids (hydrochloric acid) leads to uncontrolled chemical processes during starch hydrolysis, for example, the formation of the products of glucose reversal, including the formation of highly dangerous compounds: furfurol, methylfurfurol, and oxymethylfurfurol. The high concentration of ground grain leads to high viscosity of the reaction mass. This can lead to instability of the process, for example, to impenetrable blocks of the reaction mass, especially when the grain processing is stopped when stored without treatment, which is described in the patent RU2265364. Therefore, this technology does not lead to complete hydrolysis of wheat and rye starches, and some part of glucose is lost as a result of side reactions, which indicates that the method is not quite effective and lowers the final value of the feed molasses product.

The technological parameters of enzymatic hydrolysis given in the patent RU2265364 are doubtful. The claimed temperature of wheat starch gelation in the range 43-50°C is low and insufficient for complete erosion of starch granules because it is well known from the literature that the temperature of the start of wheat starch gelation is 58°C, and the temperature of maximum viscosity is 74°C. This means that at temperatures of 43-50°C given in the patent RU2265364, starch granules cannot completely decompose, amylose and amylopectin do not diffuse into the liquid part of solution, and the enzymatic hydrolysis of starch cannot go to the end.

As for the term "cavitation" widely used in the patent RU2265364, it is also inapplicable to hydrolysis of viscous and highly viscous media such as starch paste and carbohydrate syrup. This term can be used for media with low viscosity: fresh and salt water, milk, and other aqueous media not containing any polymeric and gelatinous compounds with variable viscosity, which exceeds the viscosity of water 345-fold for wheat starch, 230-fold for rye starch, and 880-fold for potato starch.

The technical problems solved in the proposed invention are: increasing the environmental safety and providing the stability of the process for the production of feed starch syrup from starch-containing plant raw materials, providing complete starch processing, and obtaining a product with high contents of simple carbohydrates suitable for long-term storage without further processing.

In the proposed method for the production of feed starch syrup from starch-containing plant raw materials comprising the hydromechanical treatment of the preliminarily ground starch-containing plant raw material, gelation, further liquefaction of plant starch in the presence of liquefying amylolytic enzymes, and saccharification in the presence of saccharifying amylolytic enzymes, according to the invention, at first, the cavitation treatment of water is performed in a disperser while heating water to 25-40°C due to the conversion of mechanical energy into thermal energy,
then the ground starch-containing plant raw material with at least 40% starch and with particle size of no more than 1000 µm and liquefying amylolytic enzymes are gradually added to treated water at a weight ratio of water to starch-containing raw material of 1 : 0.4-0.6, gelation and liquefaction of plant starch are performed at 58-85°C; the reaction mass is cooled to 50-60°C,
the second portion of the starch-containing raw material preliminarily IR irradiated until the loss of integrity of the grains of the starch-containing raw material is gradually added until the total weight ratio of water to starch-containing raw material becomes 1 : 0.9-1.1, liquefying amylolytic enzymes are further added, and gelation and liquefaction of plant starch are performed at 58-85°C;
saccharifying amylolytic enzymes are added to the resulting liquefied mixture, and saccharification of starch is performed.

The cavitation treatment of water at the initial stage is aimed at increasing its reactivity to destroy the water clusters. It is important to increase the reactivity of water because it is directly involved in starch hydrolysis. The proposed cavitation treatment of water makes it possible to process the starch-containing raw material without introducing acids. Water is heated to 25-40°C in a hydromechanical disperser without using any additional heating devices due to the transformation of mechanical energy into thermal energy. At a water temperature below 25°C, the introduced ground starch-containing plant raw material, for example, ground grain, agglomerates and is poorly dispersed; when the raw material is introduced at temperatures above 40°C, surface gelation of the raw material occurs (without gelation in the bulk), forming partially gelated clumps of the starch-containing raw material floating on the surface of the reaction mass.

To obtain feed additives with high contents of simple carbohydrates suitable for long-term storage, ground starch-containing plant raw materials with at least 40% starch should be used. The amount of the ground starch-containing plant raw material introduced at the first stage (at a weight ratio of water to starch-containing raw materials of 1 : 0.4-0.6) is determined by the technological parameters of the stage of gelation of plant starches.

To obtain a product with a high content of easily digestible carbohydrates suitable for long-term storage without further treatment, the concentration of starch (grain or other starch-containing plant raw material) in the reaction mass should be increased. As is known, the concentration of food molasses in aqueous medium in the production of molasses from native starch is 30-33%. At higher starch concentrations, impenetrable blocks can form at the gelation stage, and the motion of the reaction mass along the product lines and in reaction vessels stops because of high viscosity. This situation leads to a halt in the whole process, the loss of raw materials, and high material costs associated with the halt of the process and elimination of emergency. Therefore, the introduction of grain (or other starch-containing raw materials) in an amount of 40-60% of the amount of water at 40-70% starch in the raw material at the first stage does not exceed the critical starch concentrations and allows the process to be conducted without emergency. In addition, when the ground grain is introduced in the indicated amount, the amount of foam is not critical for process stability.

At the start of the process for the production of concentrated feed starch syrup, ground starch-containing plant raw materials, for example, ground grain are introduced after the treatment and heating of water. The raw material undergoes effective homogenization in an aqueous medium, forming a homogeneous reaction mass. The use of pre-ground starch-containing plant raw materials with a particle size of up to 1000 µm at the first stage is dictated by the partial loss of crystallinity of large starch granules with a size of up to 150 µm due to the mechanical treatment during grounding. The loss of crystallinity in starch granules facilitates their further processing and lowers the viscosity of the reaction mass during starch gelation.

The second portion of the IR irradiated starch-containing raw material is introduced in the reaction mass after the stage of gelation and liquefaction of the first portion of the starch-containing raw material during the preparation of feed starch syrup. If some portion of starch added at the first stage as a raw material with a very high starch content remained not completely processed, it is finally processed with the second portion of the starch-containing plant raw material, which increases the concentration of easily digestible carbohydrates (glucose, maltose) in the end product.

During the IR processing, the grain itself is partially destroyed already at the irradiation stage, and the starch present in it undergoes partial intragranular gelation under the action of radiation, which facilitates subsequent processing. Thus, the second portion of starch further successfully undergoes the most complex stage of grain processing-gelation and liquefaction in concentrated mixtures.

It is precisely this sequence of technological processes based on profound knowledge about the structure of starch and its enzymatic hydrolysis that gives a synergistic effect and leads to a positive solution of the technical problem in the preparation of concentrated carbohydrate feed additives.

In the said method for the production of feed starch syrup, the negative effects of pretreatment of the starch-containing plant raw materials are minimized during the gradual introduction of the starch-containing plant raw material in the production of feed starch syrup:
the foaming typical for the ground raw material decreases and is controlled, and the possible grain blocking in the IR irradiated raw material is eliminated.

Starch consists of two biopolymers: amylose and amylopectin, which are part of starch granules having a semi-crystalline structure. The size of starch granules varies widely from 2 to 150 µm, and many plant starches have a bimodal composition; i.e., they have large and fine granules. The large starch granules gelatinize at lower temperatures; smaller granules, at higher temperatures. Therefore, the gelation stage has a wide temperature range. For guaranteed gelation of all starch granules of the plant raw material, the temperature range 58-85°C of treatment was chosen taking into account the literature data.

As starch-containing plant raw materials, one or several types of grain raw materials without a glume selected from the group consisting of wheat, rye, barley, corn, triticale, and rice can be introduced in the process.

The grain raw materials without a glume can be introduced in both ground and preliminarily IR irradiated form.

As preliminarily IR irradiated starch-containing raw material, one or more types of leguminous raw materials selected from the group consisting of pea, kidney bean, lentil, chickpea, soybean, lupine, and everlasting pea can be introduced.

The main criteria for choosing the starch-containing plant raw materials for the production of feed starch syrup are: at least 40% starch, large-scale industrial cultivation, the absence of anti-nutrient and poisonous compounds, and no more than 10-14% cellulose-containing compounds in the shell or glume.

At the first stage, the starch-containing raw material with a higher starch content (60-77%) is used, while at the second stage, IR irradiated raw materials with lower contents of starch and other carbohydrate compounds can be used to obtain a product with a high content of simple carbohydrates that is suitable for long-term storage without additional treatment.

Before each stage of starch gelation (both at the first and second stages), proteolytic and/or cellulosolytic enzymes can be introduced simultaneously with amylolytic enzymes. The proteolytic and cellulosolytic enzymes are mainly introduced if the starch-containing plant raw material used contains more than 8% protein and over 3% cellulose grain shell, respectively. The use of proteolytic and cellulosolytic enzymes leads to complete processing of the components of plant raw materials such as proteins and cellulose grain shells, which are biopolymers consisting of glucose molecules, which increases the nutritional value of the feed starch syrup product.

The feed starch syrup produced by the said method with at least 35% easily digestible carbohydrates is also patented. The name of the product is feed starch syrup because it contains high concentrations of easily digestible carbohydrates obtained by enzymatic hydrolysis of plant starch and also other components of grain and leguminous raw materials and/or the products of their processing (proteins, amino acids, cellulose, etc.), which are valuable components of feed for productive livestock. The shelf life of feed starch syrup with 35% easily digestible carbohydrates depends on the temperature: up to 15 days at a storage temperature of+3 to +10°C, up to 7 days at a temperature of up to +20°C, and 6 months in the frozen state.

The said method for the production of feed starch syrup is implemented as follows. The examples given below are not the only possible ones, but serve to merely illustrate the proposed method.

### Example 1.

Water (30 L) at a temperature of 10-22°C is poured in a hydromechanical disperser and treated by recirculating it through the working parts of the disperser at a disperser rotor speed of 2400-3000 rpm. During this cavitation treatment of water for 25-35 min, water in the disperser is heated to 25-40°C. After this temperature was reached, ground wheat grain (15 kg) with particle size of up to 1000 µm and 71% starch in the grain is introduced in portions in the disperser for 10-15 min, while continuously performing the hydrodynamic treatment of the mixture at a disperser rotor speed of 2400-2800 rpm. After the whole amount of ground grain was introduced, the water-grain suspension is treated for 20-30 min at a disperser rotor speed of 2600-3000 rpm until the temperature of the reaction mass has reached 49-51°C. After this, the liquefying amylolytic enzyme (alpha-amylase) Brenn Zyme HL 120L (12 g) is added. The amount of enzymes (in this and subsequent examples) depends on their initial activity indicated in the certificate and on the amount of the substance which experiences the action of enzyme: starch, protein, cellulose. The certificate also indicates the temperature range in which the enzyme shows the highest activity, which determines the process temperature. The processed water-grain mass is then heated to 85°C for complete erosion of small starch grains at a disperser rotor speed of 2800-3200 rpm for 35-45 min. During the hydromechanical processing of the reaction mass, the temperature gradually increases, and the viscosity of the water-grain suspension increases at 57-65°C and then decreases at 78-80°C, which is recorded by the ammeter on the control panel of the disperser. This change in the viscosity suggests gelation of plant starch when the viscosity of the reaction mass initially increases and liquefaction under the action of enzymes when the viscosity decreases. After that, the reaction mass is cooled to 50-60°C, and the second portion of the starch-containing raw material, namely, IR-irradiated wheat grain (15 kg) is gradually introduced. The preliminary IR treatment of whole wheat grains with 12-16% natural moisture is performed at wavelengths of 900-1100 Hz for 60-90 s at an IR radiation flux density of 20-23 kW/m² until the grain integrity is lost. The IR irradiated grain is introduced for 20-25 min at a disperser rotor speed of 2800-3200 rpm. After this, the liquefying amylolytic enzyme (alpha-amylase) Brenn Zyme HL 120L (12 g) is introduced. Then the reaction mass is heated to 85°C at a disperser rotor speed of 3200-3600 rpm and kept at this temperature for 8-10 min for gelation and liquefaction of the second portion of starch. After this, the reaction mixture is cooled to 65°C, and the saccharifying amylolytic enzyme Brenn Zyme GAF-T HC (gluco-amylase) (31 g) is added. The mixture is kept at 60-65°C and a disperser rotor speed of 2400-2600 rpm for 3 or 4 h. This procedure gives a finished product with at least 35% easily digestible carbohydrates (glucose and maltose). The storage time is 15 days at a temperature from +5 to +7°C.

### Example 2.

The first part of the starch-containing raw material - ground corn grain (15 kg) with particle size of up to 1000 µm and 74% starch in the grain - is introduced and processed as described in Example 1. The liquefying amylolytic enzyme (alpha-amylase) Brenn Zyme HL 120L (12.5 g) is introduced. During the hydromechanical processing of the reaction mass, the temperature increases. At the start of the treatment, the viscosity of the water-grain suspension increases at 69-72°C and then decreases at 79-81°C, which is recorded with an ammeter on the control panel of the disperser. After that, the reaction mass is cooled to 50-60°C, and the second portion of the IR irradiated starch-containing leguminous raw material, namely, peas (15 kg) with 44% starch is introduced. The preliminary IR irradiation of whole peas with 12-16% natural moisture is performed at wavelengths of 900-1100 Hz for 80-140 s at an IR flux density of 20-23 kW/m² until the integrity of the raw material is lost. The IR irradiated raw material is introduced for 20-25 min at a disperser rotor speed of 2800-3200 rpm. After this, the liquefying amylolytic enzyme Brenn Zyme HL 120L (alpha-amylase) (7.4 g) and proteolytic enzyme Protosubtilin (6 g) are introduced. Then the reaction mass is heated to 85°C at a disperser rotor speed of 3200-3600 rpm and kept at this temperature for 8-10 min to let it undergo the gelation and liquefaction stages. After this, the reaction mixture is cooled to 65°C, the saccharifying amylolytic enzyme Brenn Zyme GAF-T HC (glucoamylase) (25.8 g) is introduced, and the mixture is maintained at 60-65°C for 3 or 4 h at a disperser rotor speed of 2400-2600 rpm. This gives a product with at least 35% easily digestible carbohydrates (total content). The storage time is 7 days at a temperature of 13-15°C.

### Example 3.

The feed starch syrup is produced by the procedure described in Example 1, with the following differences. At the first stage, the ground rice grain (15 kg) with a particle size of up to 1000 µm and 77% starch in the grain is introduced in the disperser for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, IR irradiated wheat grain (15 kg) with 71% starch is used, liquefaction is performed under the action of the liquefying amylolytic enzyme (alpha-amylase) Brenn Zyme HL 120L (12.0 g) at a disperser rotor speed of 3400-3600 rpm, and saccharification is performed in the presence of the saccharifying amylolytic enzyme Brenn Zyme GAF-T HC (glucoamylase) (32.3 g) at a disperser rotor speed of 2800-3000 rpm. This gives a product with a total content of easily digestible carbohydrates of at least 35%.

### Example 4.

The feed starch syrup is produced by the procedure described in Example 1, with the following differences. At the first stage, the ground rice grain (15 kg) with a particle size of up to 1000 µm and 77% starch in the grain is introduced in the disperser for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, the IR irradiated corn grain (15 kg) with 74% starch is used, liquefaction is performed under the action of the liquefying amylolytic enzyme (alpha-amylase) Brenn Zyme HL 120L (12.5 g) at a disperser rotor speed of 3400-3600 rpm, and saccharification is performed in the presence of the saccharifying amylolytic enzyme Brenn Zyme GAF-T HC (glucoamylase) (33.0 g) at a disperser rotor speed of 3000-3200 rpm. This gives a product with a total content of easily digestible carbohydrates of at least 35%.

### Example 5.

The feed starch syrup is produced by the procedure described in Example 1, with the following differences. At the first stage, a grain mixture (18 kg) with a particle size of up to 1000 µm containing ground corn grain (9 kg) with 74% starch and ground wheat grain (9 kg) with 71% starch is introduced in the disperser for 14-20 min at a disperser rotor speed of 2800-3200 rpm, and liquefaction is performed under the action of the liquefying amylolytic enzyme (alpha-amylase) Brenn Zyme HL 120L (15.0 g) at a disperser rotor speed of 3400-3600 rpm. At the second stage, IR irradiated corn grain (9 kg) with 74% starch is used, liquefaction is performed under the action of the liquefying amylolytic enzyme (alpha-amylase) Brenn Zyme HL 120L (7.5 g) at a disperser rotor speed of 3400-3600 rpm, and saccharification is performed in the presence of the saccharifying amylolytic enzyme Brenn Zyme GAF-T HC (glucoamylase) (29.1 g) at a disperser rotor speed of 3000-3200 rpm. This gives a product with a total content of easily digestible carbohydrates of at least 35%.

### Example 6.

The feed starch syrup is produced by the procedure described in Example 1, with the following differences. At the first stage, the ground rice grain (18 kg) with a particle size of up to 1000 µm and 77% starch in the grain is introduced for 14-20 min at a disperser rotor speed of 3200-3400 rpm, and liquefaction is performed under the action of the liquefying amylolytic enzyme (alpha-amylase) Brenn Zyme HL 120L (15.6 g) at a disperser rotor speed of 3400-3600 rpm. At the second stage, IR irradiated rice grain (9 kg) with 77% starch is used, liquefaction is performed with the liquefying amylolytic enzyme (alpha-amylase) Brenn Zyme HL 120L (7.8 g) at a disperser rotor speed of 3400-3600 rpm, and saccharification is performed in the presence of the saccharifying amylolytic enzyme Brenn Zyme GAF-T HC (glucoamylase) (30.3 g) at a disperser rotor speed of 3000-3200 rpm. This gives a product with a total content of easily digestible carbohydrates of at least 35%.

### Example 7.

The feed starch syrup is produced by the procedure described in Example 1, with the following differences. At the first stage, the ground barley grain (15 kg) with a particle size of up to 1000 µm and 62% starch in the grain is introduced in a disperser for 10-15 min at a disperser rotor speed of 2600-3000 rpm, and liquefaction is performed under the action of the liquefying amylolytic enzyme (alpha-amylase) Brenn Zyme HL 120L (10.5 g) at a disperser rotor speed of 3000-3200 rpm in the presence of the cellulolytic enzyme Cellollux (2.0 g). At the second stage, IR irradiated barley grain (15 kg) with 62% starch is used, liquefaction is performed under the action of the liquefying amylolytic enzyme (alpha-amylase) Brenn Zyme HL 120L (10.5 g) at a disperser rotor speed of 3200-3400 rpm in the presence of the cellulolytic enzyme Cellollux (2.0 g), and saccharification is performed in the presence of the saccharifying amylolytic enzyme Brenn Zyme GAF-T HC (glucoamylase) (27.1 g) at a disperser rotor speed of 3000-3200 rpm. This gives a product with a total content of easily digestible carbohydrates of at least 35%.

### Example 8.

The feed starch syrup is produced by the procedure described in Example 1, with the following differences. At the first stage, the ground corn grain (18 kg) with a particle size of up to 1000 µm and 74% starch in the grain is introduced for 10-15 min at a disperser rotor speed of 2800-3200 rpm, and liquefaction is performed under the action of the liquefying amylolytic enzyme (alpha-amylase) Brenn Zyme HL 120L (15.0 g) at a disperser rotor speed of 3200-3400 rpm. At the second stage, IR irradiated triticale grain (12 kg) with 65% starch is used, liquefaction is performed under the action of the liquefying amylolytic enzyme (alpha-amylase) Brenn Zyme HL 120L (8.8 g) at a disperser rotor speed of 3400-3600 rpm, and saccharification is performed in the presence of the saccharifying amylolytic enzyme Brenn Zyme GAF-T HC (glucoamylase) (30.8 g) at a rotor speed of 3000-3200 rpm. This gives a product with a total content of easily digestible carbohydrates of at least 35%.

### Example 9.

The feed starch syrup is produced by the procedure described in Example 1, with the following differences. At the first stage, the ground rice grain (18 kg) with a particle size of up to 1000 µm and 77% starch in the grain is introduced for 15-20 min in a disperser at a rotor speed of 2800-3000 rpm, and liquefaction is performed under the action of the liquefying amylolytic enzyme (alpha-amylase) Brenn Zyme HL 120L (15.6 g) at a disperser rotor speed of 3200-3400 rpm. At the second stage, IR irradiated barley grain (12 kg) with 62% starch is used, liquefaction is performed under the action of the amylolytic enzyme (alpha-amylase) Brenn Zyme HL 120L (8.4 g) at a disperser rotor speed of 3400-3600 rpm, and saccharification is performed in the presence of the saccharifying amylolytic enzyme Brenn Zyme GAF-T HC (glucoamylase) (30.2 g) at a disperser rotor speed of 3000-3200 rpm. This gives a product with a total content of easily digestible carbohydrates of at least 35%.

### Example 10.

The feed starch syrup is produced by the procedure described in Example 1, with the following differences. At the first stage, the ground wheat grain (9 kg) with a particle size of up to 1000 µm and 71% starch in the grain is introduced for 10-12 min in a disperser at a rotor speed of 2400-2800 rpm, and liquefaction is performed under the action of the liquefying amylolytic enzyme (alpha-amylase) Brenn Zyme HL 120L (7.2 g) at a disperser rotor speed of 3000-3200 rpm. At the second stage, IR irradiated corn grain (24 kg) with 74% starch is used, liquefaction is performed under the action of the liquefying amylolytic enzyme (alpha-amylase) Brenn Zyme HL 120L (20.0 g) at a disperser rotor speed of 3400-3600 rpm, and saccharification is performed in the presence of the saccharifying amylolytic enzyme Brenn Zyme GAF-T HC (glucoamylase) (35.2 g) at a disperser rotor speed of 3200-3400 rpm. This gives a product with a total content of easily digestible carbohydrates of at least 35%.

### Example 11.

The feed starch syrup is produced by the procedure described in Example 1, with the following differences. At the first stage, the ground wheat grain (18 kg) with a particle size of up to 1000 µm and 71% starch in the grain is introduced for 10-15 min at a rotor speed of 2800-3000 rpm, and liquefaction is performed under the action of the liquefying amylolytic enzyme (alpha-amylase) Brenn Zyme HL 120L (14.4 g) at a disperser rotor speed of 3200-3400 rpm. At the second stage, IR irradiated rye grain (15 kg) with 64% starch is used, liquefaction is performed under the action of the liquefying amylolytic enzyme (alpha-amylase) Brenn Zyme HL 120L (10.8 g) at a disperser rotor speed of 3400-3600 rpm, and saccharification is performed in the presence of the saccharifying amylolytic enzyme Brenn Zyme GAF-T HC (glucoamylase) (32.6 g) at a disperser rotor speed of 3000-3200 rpm. This gives a product with a total content of easily digestible carbohydrates of at least 35%.

### Example 12.

The feed starch syrup is produced by the procedure described in Example 1, with the following differences. At the first stage, the ground wheat grain (18 kg) with a particle size of up to 1000 µm and 71% starch in the grain is introduced for 10-15 min at a disperser rotor speed of 2800-3000 rpm, and liquefaction is performed under the action of the liquefying amylolytic enzyme (alpha-amylase) Brenn Zyme HL 120L (14.4 g) at a disperser rotor speed of 3200-3300 rpm. At the second stage, IR irradiated peas (12 kg) with 44% starch is used, liquefaction is performed under the action of the liquefying amylolytic enzyme (alpha-amylase) Brenn Zyme HL 120L (5.9 g) at a disperser rotor speed of 3400-3600 rpm, the proteolytic enzyme Protosubtilin (5 g) is added before the gelation stage, and saccharification is performed in the presence of the saccharifying amylolytic enzyme Brenn Zyme GAF-T HC (glucoamylase) (32.6 g) at a disperser rotor speed of 3000-3200 rpm. This gives a product with a total content of easily digestible carbohydrates of at least 35%.

### Example 13.

The feed starch syrup is produced by the procedure described in Example 1, with the following differences. At the first stage, the ground corn grain (18 kg) with a particle size of up to 1000 µm and 74% starch in the grain is introduced for 15-20 min at a disperser rotor speed of 3000-3200 rpm, and liquefaction is performed under the action of the liquefying amylolytic enzyme (alpha-amylase) Brenn Zyme HL 120L (15.0 g) at a disperser rotor speed of 3200-3300 rpm. At the second stage, the IR irradiated leguminous mixture (15 kg) containing soybeans (7.5 kg) with 26 % carbohydrates and pea beans (7.5 kg) with 44% starch is used, liquefaction is performed under the action of the liquefying amylolytic enzyme (alpha-amylase) Brenn Zyme HL 120L (9.1 g) a t a disperser rotor speed of 3000-3100 rpm, the proteolytic enzyme Protosubtilin (8 g) is added before the gelation stage, and saccharification is performed in the presence of the saccharifying amylolytic enzyme Brenn Zyme GAF-T HC (glucoamylase) (31.2 g) at a disperser rotor speed of 3000-3200 rpm. This gives a product with a total content of easily digestible carbohydrates of at least 35%.

### Example 14.

The feed starch syrup is produced by the procedure described in Example 1, with the following differences. At the first stage, the ground corn grain (15 kg) with a particle size of up to 1000 µm and 74% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (12.5 g) at a disperser rotor speed of 3000-3300 rpm. At the second stage, IR irradiated chickpea beans (15 kg) with 47% starch are used, liquefaction is performed under the action of the liquefying amylolytic enzyme (alpha-amylase) Brenn Zyme HL 120L (7.9 g) at a disperser rotor speed of 3400-3600 rpm, and saccharification is performed in the presence of the saccharifying amylolytic enzyme Brenn Zyme GAF-T HC (glucoamylase) (26.7 g) at a disperser rotor speed of 3000-3200 rpm. This gives a product with a total content of easily digestible carbohydrates of at least 35%.

### Example 15.

The feed starch syrup is produced by the procedure described in Example 1, with the following differences. At the first stage, the ground rice grain (15 kg) with a particle size of up to 1000 µm and 77% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, IR irradiated kidney beans (15 kg) with 45% starch are used, liquefaction is performed under the action of the liquefying amylolytic enzyme (alpha-amylase) Brenn Zyme HL 120L (7.6 g) at a disperser rotor speed of 3400-3600 rpm, and saccharification is performed in the presence of the saccharifying amylolytic enzyme Brenn Zyme GAF-T HC (glucoamylase) (26.0 g) at a disperser rotor speed of 3000-3200 rpm. This gives a product with a total content of easily digestible carbohydrates of at least 35%.

### Example 16.

The feed starch syrup is produced by the procedure described in Example 1, with the following differences. At the first stage, the ground rice grain (15 kg) with a particle size of up to 1000 µm and 77% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, IR irradiated lentil beans (15 kg) with 45% starch are used, liquefaction is performed under the action of the liquefying amylolytic enzyme (alpha-amylase) Brenn Zyme HL 120L (7.6 g) at a disperser rotor speed of 3400-3600 rpm, and saccharification is performed in the presence of the saccharifying amylolytic enzyme Brenn Zyme GAF-T HC (glucoamylase) (26.7 g) at a disperser rotor speed of 3000-3200 rpm. This gives a product with a total content of easily digestible carbohydrates of at least 35%.

### Example 17.

The feed starch syrup is produced by the procedure described in Example 1, with the following differences. At the first stage, the ground rice grain (18 kg) with a particle size of up to 1000 µm and 77% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (15.6 g) at a disperser rotor speed of 3000-3300 rpm. At the second stage, an IR irradiated legume mixture (15 kg) containing soybeans (7.5 kg) with 26% carbohydrates and lupine beans (7.5 kg) with 46% starch is used, liquefaction is performed under the action of the liquefying amylolytic enzyme (alpha-amylase) Brenn Zyme HL 120L (9.3 g) at a disperser rotor speed of 3400-3600 rpm, and saccharification is performed in the presence of the saccharifying amylolytic enzyme Brenn Zyme GAF-T HC (glucoamylase) (32.1 g) at a disperser rotor speed of 3000-3200 rpm. This gives a product with a total content of easily digestible carbohydrates of at least 35%.

### Example 18.

The feed starch syrup is produced by the procedure described in Example 1, with the following differences. At the first stage, the ground rice grain (15 kg) with a particle size of up to 1000 µm and 77% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, IR irradiated lupine beans (15 kg) with 46% starch are used, liquefaction is performed under the action of the liquefying amylolytic enzyme (alpha-amylase) Brenn Zyme HL 120L (7.8 g) at a disperser rotor speed of 3400-3600 rpm, and saccharification is performed in the presence of the saccharifying amylolytic enzyme Brenn Zyme GAF-T HC (glucoamylase) (26.9 g) at a disperser rotor speed of 3000-3200 rpm. This gives a product with a total content of easily digestible carbohydrates of at least 35%.

### Example 19.

The feed starch syrup is produced by the procedure described in Example 1, with the following differences. At the first stage, the ground rice grain (15 kg) with a particle size of up to 1000 µm and 77% starch in the grain is introduced for 10-15 min at a disperser rotor speed of 2600-3000 rpm, and liquefaction is performed under the action of the liquefying amylolytic enzyme (alpha-amylase) Brenn Zyme HL 120L (13.0 g) at a disperser rotor speed of 3000-3300 rpm. At the second stage, IR irradiated everlasting pea beans (15 kg) with 45% starch are used, liquefaction is performed under the action of the liquefying amylolytic enzyme (alpha-amylase) Brenn Zyme HL 120L (7.6 g) at a disperser rotor speed of 3400-3600 rpm, and saccharification is performed in the presence of the saccharifying amylolytic enzyme Brenn Zyme GAF-T HC (glucoamylase) (26.7 g) at a disperser rotor speed of 3000-3200 rpm. This gives a product with a total content of easily digestible carbohydrates of at least 35%.

### Example 20.

The feed starch syrup is produced by the procedure described in Example 1, with the following differences. At the first stage, the ground wheat grain (15 kg) with a particle size of up to 1000 µm and 71% starch in the grain is introduced for 10-20 min at a disperser rotor speed of 2800-3000 rpm, and liquefaction is performed under the action of the liquefying amylolytic enzyme (alpha-amylase) Brenn Zyme HL 120L (12.0 g) at a disperser rotor speed of 3000-3200 rpm. At the second stage, IR irradiated chickpea beans (18 kg) with 47% starch are used, liquefaction is performed under the action of the liquefying amylolytic enzyme (alpha-amylase) Brenn Zyme HL 120L (9.5 g) at a disperser rotor speed of 3400-3600 rpm, and saccharification is performed in the presence of the saccharifying amylolytic enzyme Brenn Zyme GAF-T HC (glucoamylase) (27.9 g) at a disperser rotor speed of 3200-3400 rpm. This gives a product with a total content of easily digestible carbohydrates of at least 35%.

### Example 21.

The feed starch syrup is produced by the procedure described in Example 1, with the following differences. At the first stage, the ground wheat grain (15 kg) with a particle size of up to 1000 µm and 71% starch in the grain is introduced for 15-20 min at a disperser rotor speed of 2800-3000 rpm, and liquefaction is performed under the action of the liquefying amylolytic enzyme (alpha-amylase) Brenn Zyme HL 120L (12.0 g) at a disperser rotor speed of 3000-3200 rpm. At the second stage, IR irradiated chickpea beans (18 kg) with 47% starch are used, liquefaction is performed under the action of the liquefying amylolytic enzyme (alpha-amylase) Brenn Zyme HL 120L (9.5 g), the protolytic enzyme Protosubtilin (11 g) and cellulolytic enzyme Cellollux (4.0 g) are added before the gelation stage at a disperser rotor speed of 3400-3600 rpm, and saccharification is performed in the presence of the saccharifying amylolytic enzyme Brenn Zyme GAF-T HC (glucoamylase) (27.9 g) at a disperser rotor speed of 3200-3400 rpm. This gives a product with a total content of easily digestible carbohydrates of at least 35%.

The feed starch syrup obtained by the method described in the examples had a shelf life of up to 15 days at a storage temperature from +3 to +10°C and up to 7 days at a temperature of up to +20°C.

The present invention makes it possible to increase the environmental safety and stability of the process, ensures complete starch processing, and affords a product with a high content of easily digestible carbohydrates suitable for long-term storage without additional treatment.

## Claims

1. A method for the production of feed starch syrup from starch-containing plant raw material comprising:
hydrodynamic treatment of a mixture of the preliminarily ground starch-containing plant raw material and water,
gelation and liquefaction of plant starch in the presence of liquefying amylolytic enzymes, and
starch saccharification in the presence of amylolytic saccharifying enzymes,
**characterized in that** at first the cavitation treatment of water is performed in a disperser while heating water to 25-40 °C, the ground starch-containing plant raw material with at least 40% starch in it and with a particle size of no more than 1000 µm and liquefying amylolytic enzymes are gradually introduced in treated water at a weight ratio of water to starch-containing raw material of 1 : 0.4-0.6,
gelation and liquefaction of the plant starch are performed at 58-85°C; the reaction mass is cooled to 50-60°C, and
the second part of the starch-containing raw material preliminarily IR irradiated until the loss of integrity of the grains of the starch-containing raw material is gradually introduced in an amount sufficient for reaching the total weight ratio of water to starch-containing raw material of 1 : 0.9-1.1, liquefying amylolytic enzymes are additionally introduced, and gelation and liquefaction of the plant starch are performed at 58-85°C;
saccharifying amylolytic enzymes are introduced in the resulting liquefied mixture, and starch saccharification is performed.

2. The method of claim 1, wherein the starch-containing plant raw material is one or more types of grain raw material without a glume selected from the group containing wheat, rye, barley, corn, triticale, and rice.

3. The method of claim 2, wherein the grain raw material without a glume is introduced as the ground starch-containing plant raw material.

4. The method of claim 2, wherein the grain raw material without a glume is introduced as the preliminarily IR irradiated starch-containing raw material.

5. The method of claim 1, wherein the preliminarily IR irradiated starch-containing raw material is one or more types of leguminous raw materials selected from the group containing pea, kidney bean, lentil, chickpea, soybean, lupine, and everlasting pea.

6. The method of any claim 1-5, wherein proteolytic enzymes are further introduced before the gelation stage.

7. The method of any claim 1-6, wherein cellulosolytic enzymes are further introduced before the gelation stage.

8. Feed starch syrup produced by the method of any claim 1-7, which contains at least 35% of easily digestible carbohydrates.
